Europäisches Patentamt

European Patent Office

Office européen des brevets

(11) Publication number: **0 323 909**
**A1**

## EUROPEAN PATENT APPLICATION

(21) Application number: **89300100.8**

(22) Date of filing: **06.01.89**

(51) Int. Cl.⁴: **G 01 N 33/566**
**G 01 N 33/569,**
**G 01 N 33/545, C 12 Q 1/06**
**// C12Q1/68**

(30) Priority: **07.01.88 GB 8800292**

(43) Date of publication of application:
**12.07.89 Bulletin 89/28**

(84) Designated Contracting States:
**BE CH DE FR GB IT LI LU NL SE**

(71) Applicant: **INTERNATIONAL INSTITUTE OF CELLULAR & MOLECULAR PATHOLOGY**
**Catholic University of Louvain 75 Avenue Hippocrate**
**B-1200 Brussels (BE)**

(72) Inventor: **Cambiaso, Cesar Lorenzo**
**Rue F. Cloetens, 13**
**B-1950 Kraainem (BE)**

**Collet-Cassart, Daniel**
**16 Avenue de Mimosa**
**B-1950 Kraainem (BE)**

(74) Representative: **Pennant, Pyers**
**Stevens, Hewlett & Perkins 5 Quality Court Chancery Lane**
**London, WC2A 1HZ (GB)**

The title of the invention has been amended (Guidelines for Examination in the EPO, A-III, 7.3).

(54) **Immunological assay method.**

(57) An agglutination assay for an analyte which is a member of a specific binding pair involves the use of a) suspension of particles coated with a member of the specific binding pair, and if not already present b) the complementary member of the specific binding pair. The method is characterized by including a lectin which binds to a saccharide wherein either the analyte or one of reagents a) and b) carries the saccharide. The assay may be performed either in a direct mode, in which the analyte causes agglutination, or in an inhibition mode, in which the analyte inhibits agglutination. The analyte may be an antigen or hapten or an antibody or may comprise bacteria.

EP 0 323 909 A1

## Description

## ASSAY METHOD

Introduction

Lectins belong to a category of proteins which react with carbohydrate. They are characterized by their multivalency and their agglutinating activity towards various living cells.

The agglutinating activity of these highly specific carbohydrate-binding molecules is usually inhibited by a simple monosaccharide, but for some lectins di, tri and even polysaccharides are required.

Lectins are isolated from a wide variety of natural sources including seeds, plantroots and bark, fungi, bacteria, seeweed and sponges, molluscs, fish eggs, body fluids of invertebrates and lower vertebrates and from mamalian cell membranes. The precise physiological role of lectins in nature is still unknown but a large body of evidence indicates that lectins are adapted for a variety of cell surface and intracellular functions, for extacellular material organization, for recognition functions, etc.

Lectins have proved to be very valuable in a wide variety of in vitro applications
- blood grouping and erythrocyte polyagglutination studies
- mitogenic stimulation of lymphocytes
- lymphocyte subpopulation studies
- fractionation of cells and other particles
- isolation, purification and structural studies of carbohydrate containing molecules
- histochemical studies of normal and pathological conditions

Information about lectins can be found in:
- Sharon and LiS Science, 177, 949 (1972)
- LiS and Sharon Ann. Rev. biochem. 42, 541 (1973)
- Sharon "Advances in Immunology" Dixon and Kunkel ed., Academic Press London and New York P213 (1983)
- Barondes Ann. Rev. Biochem. 50, 207 (1981)
- Bog-Hansen "Lectins: Biology, Biochemistry, Clinical Biochemistry" W. de Gruyter - Berlin Volumes 1 to V.

This invention concerns the use of lectins in agglutination assays, and arises from the idea that the agglutinating power of lectins is of particular interest for particle assays. As is well known, in agglutination assays particulate materials such as polystyrene (generally referred to as latex) are coated with an assay reagent. Upon incubation with the assay sample and any other desired reagents, the particles may become agglutinated, to an extent related to the concentration of an analyte in the sample. Various different assay systems are known and described in the literature, of which two are worth mentioning at this stage. In direct assays, the analyte causes agglutination, the extent of which is therefore generally directly proportional to the concentration of the analyte in the sample. In inhibition assays, the analyte inhibits agglutination, the extent of which is accordingly generally inversely proportional to the concentration of the analyte in the sample. In many systems, the assay can be operated either in the direct mode or in the inhibition mode to measure either of two different analytes. The agglutination assays with which this invention is concerned may in general be operated either in the direct or the inhibition mode.

In one aspect, the invention provides a method of assaying for an analyte in a sample, which analyte is a member of a specific binding pair, by the use of:

a) a suspension of particles coated with a member of the specific binding pair,

b) if not present in a), the complementary member of the specific binding pair to the analyte, and

c) a lectin which binds to a saccharide, wherein either the analyte or one reagents a) and b) carries the saccharide,

which method comprises incubating the sample in any order with reagents a), c) and if present b), and observing agglutination of the particles to an extent related to the concentration of the analyte in the sample.

The analyte is a member of a specific binding pair. An important example of a specific binding pair is an antigen or hapten and its associated antibody. In this case, the assay relies on the immune reaction between the two, and is called an immunoassay. Another example of a specific binding pair are two complementary single-stranded oligonucleotides or complementary single strands of DNA and/or RNA. Various other specific binding pairs are well known to workers in the field. Subject to the requirement that it be a member of a specific binding pair, the nature of the analyte is not critical to this aspect of the invention. The other member of the specific binding pair, used in the assay, is said to be complementary to the analyte. An analogue of the analyte is a substance, often a derivative of the analyte, which binds to the specific binding partner in a similar way to the analyte, and therefore competes with the analyte for binding to the specific binding partner.

In another aspect, the invention provides a method of assaying for bacteria in a sample by the use of particles coated with a glycoprotein or a saccharide, which method comprises incubating a suspension of the particles with the sample, in the presence of a lectin which binds to the glycoprotein or saccharide, and observing agglutination of the particles to an extent related to the bacterial concentration of the sample.

The nature of sample is not critical to the invention. Often, it will be a body fluid such as serum or plasma.

The invention involves the use of a suspension of particles coated with an assay reagent. These particles are of microscopic or sub-microscopic size, i.e. generally smaller than 15 microns and often of the order of a few microns or sub-micron in size. Polystyrene particles of such sizes are commercially available, and suspensions thereof are generally known as latex. It is known to bind assay reagents to microscopic particulate materials such as latex, and techniques for doing this will not be described here. In some assay systems, it may be convenient to use magnetically attractable particles, and these are also well known and commercially available.

Some agglutination assay systems involve the use of two different particulate reagents. Generally, the reagents are immobilised on uniform particles of two different sizes. Or one set of particles may be made magnetically attractable, while the other is not. The methods of this invention encompass assays involving the use of two different particulate reagents.

The assay methods of the invention involve use of a lectin which binds to a saccharide or a glycoprotein. In some cases, the lectin, or the saccharide or glycoprotein, may be present as a component of the analyte, and in such cases the choice of the complementary reagent (saccharide or glycoprotein, or lectin) is predetermined. In other assay systems, both the lectin and the saccharide or glycoprotein are present as added assay reagents. Provided that they bind to one another, the choice of these reagents is not critical to the invention. While monosaccharides and disaccharides are possible in some instances, the saccharide will generally be a polysaccharide.

The final step of the method involves observing agglutination of the particles. This can be done by particle counting, nephelometry, turbidimetry, or even simple visual estimation of agglutination. The extent of agglutination is preferably determined by a particle counting technique such as the IMPACT (trademark) diagnostic technique. This system involves counting particle numbers in a defined volume of liquid. The particles are of uniform size, and the instrument is calibrated to count only particles of approximately that size, so that assemblies of two or more particles formed by agglutination are rejected and not counted.

As noted above, the assay methods of this invention may generally be performed in a direct or a inhibition mode. The extent of agglutination is related to the concentration of analyte in the sample, but it should be understood that the relationship may be either direct or inverse, and is not necessarily linear. It is generally necessary to assay a number of standard samples of known analyte concentration spanning the range expected to be found in practice, and to use the results to generate a graph of agglutination against analyte concentration, off which the analyte concentration of unknown samples can be read.

There follow descriptions of five assay sytems according to the invention. The analyte is:
- a member of an immune binding pair in systems one to three;
- a polynucleotide in system four; and
- bacteria in system five.

1. Use of lectin as a second antibody.

Latex particles are coated with an antigen or a hapten protein conjugate. In a first step, a specific antibody is added: the antibody reacts with the latex and eventually agglutinates if its concentration is high enough. In a second step, which may be performed with or subsequent to the first step, a lectin, specific for the natural sugars of the antibody or for sugars artifically coupled to the antibody, is added and agglutinates the particles or reinforces the existing agglutination.

In the direct mode, this system can serve for antibody determination (serology).

In the inhibition mode, the system can serve to assay antigens or haptens: in this case the hapten or antigen of interest has to react first with the antibody in order to decrease the agglutinating level of the reaction.

2. Use of lectin as a reversible agglutinator.

Several experiments in the laboratory have showed that concentration of latex particles by a magnetic device (for magnetically attractable particles) or by centrifugation allows improvement in sensitivity of the agglutination reaction. The improvements were probably due to the locally increased particle numbers caused by centrifugation or concentration with the magnetic device.

This assay system makes use of this effect in lectin-saccharide interaction. In a first step, latex particles coated both with antibody and with saccharide are incubated with antigen to be assayed and a suitable lectin. It is thought that agglutination of particles by the lectin increases the number of efficient collisions of latex particles for antigen-antibody agglutination. In a second step the specific sugar (to which the lectin binds) is added to the reaction medium in order to destroy all agglutinating bonds due to lectin-sugar reactions: only agglutinating bonds due to antigen-antibody reactions remain.

It is possible to use this system in a direct or an inhibition mode. Also, in order to assay for antibody, it is possible to coat the latex with antigen plus saccharide.

3. Use of lectin to enhance avidity of antibodies.

As avidity is due to multifunctionalty of antibodies (IgM antibodies are generally of low affinity but of high avidity), it is likely that the multiple binding sites of lectins permit the prepartion of IgG-lectin complexes in which the IgG/lectin ratio is greater than one. To prepare the complexes, it may be necessary or advantageous to couple a sugar tail to the IgG antibodies. The lectin-antibody complexes so obtained are expected to display stronger agglutinating power towards latex high-antigen particles than the free form of the antibody. Note that this system resembles 1 above, except that the lectin-antibody complex is formed before, rather than after, incubation with the sample containing the analyte.

4. Use of lectin for DNA probe.

Latex particles coated with DNA fragments can react with complementary DNA fragments previously coupled to saccharides. This activated latex is agglutinable by lectins specific for the chosen saccharides. Inhibition of agglutination by complementary DNA from biological samples is possible and proportional to its concentration.

## 5. Assays for bacteria.

It is possible to use lectin-bacterial protein reactions and bacterial lectin-saccharide reactions to specifically detect bacteria by means of a particle agglutination assay. Two possible reaction schemes are:-

i) latex particles are coated with bacterial glycoprotein or bacterial polysaccharide; a specific agglutination is then possible by means of one or more suitable lectins. The assay is operated in the inhibition mode by having as analyte bacteria bearing the right polysaccharide.

ii) Latex particles are again coated with glycoproteins or poylsaccharides. These are specifically agglutinatable by bacteria bearing a lectin specific for the glycoprotein or polysaccharide coating.

The following examples illustrate the invention.

## Example 1

This reaction scheme illustrates how it is possible to detect bacteria in biological fluids. $IgA_1$ is a glycoprotein specific to a bacterium or to a defined group of bacteria. A latex coated with glycoprotein is agglutinable with a specific lectin, in this case Jacalin. The presence of bacteria bearing the same glycoprotein may be detected by their ability to inhibit the agglutination.

Practically, the test is performed as follows: 30 µl of inhibitor, in this case galactose for example, is mixed together with 30 µl of a Jacalin solution and th $IgA_1$ coated latex. After 30 minutes at 37°C of incubation in an IMPACT machine, the reaction is stopped by dilution with buffer and the particles are counted. In the absence of inhibitor one obtains a maximum of agglutination reaction. If increasing amounts of inhibitor are added, increasing inhibitions occur (the number of free particles increases).

Replacement of the galactose by a sample possibly containing bacteria bearing a saccharide (such as galactose) reactive with Jacalin, permits the test to be used as an assay for the bacteria.

## Example 2

The test performed as follows in the IMPACT machine: 30 µl of various concentrations of Concanavalin A are mixed with 30 µl of latex coated with Toxoantigens. The mixture is incubated for 30 min at 37°C, diluted and then the particles are counted. An agglutination curve directly proportional to the Concanavalin A concentration is obtained. If in the dilution buffer alpha-methylglucoside is added (10 mM) all agglutinations are destroyed. If the test is performed in the presence of a sample containing IgM antibodies, a proportion, depending on the IgM concentration, of the agglutination remains after alpha-methylglucoside addition. Thus the test can be used as an IgM assay.

## Example 3

IgM anti-Brucella.

30 µl of latex coated with Brucella lipopolysaccharide (LPS) was mixed with various dilutions (30 µl) of a positive serum for IgM anti-Brucella. Then 30 µl of buffer (GBS-BSA 1 %) or of buffer containing 125 µg Concanavalin A/mL was added. After 10 minutes incubations on the vortexing tray the reaction milieus were diluted and counted in the IMPACT machine. In the presence of Concanavalin A a reinforcement of the agglutination curve by a factor of two was observed.

## Example 4

IgM anti-Toxo

30 µl of Toxoantigens coated latex was mixed with various dilutions of an IgM positive serum (1:1 to 1:512 in GBS-BSA buffer). Then 30 µl of GBS-BSA buffer or of GBS-BSA buffer containing 125 µg Concanavalin A/mL were added. After 15 minutes incubation on a vortexing tray the reaction milieus were diluted and counted on the IMPACT machine. In the presence of Concanavalin A an improvement by a factor of 16 was observed for the agglutination curve.

## Claims

1. A method of assaying for an analyte in a sample, which analyte is a member of a specific binding pair, by the use of :

a) a suspension of particles coated with a member of the specific binding pair,

b) if not present in a), the complementary member of the specific binding pair to the analyte, and

c) a lectin which binds to a saccharide, wherein either the analyte or one of reagents a) and b) carries the saccharide, which method comprises incubating the sample in any order with reagents a), c), and if present b), and observing agglutination of the particles to an extent related to the concentration of the analyte in the sample.

2. A method as claimed in claim 1, wherein the analyte is an antibody carrying the saccharide, and reagent a) is a suspension of particles coated with an antigen to the antibody.

3. A method as claimed in claim 1, wherein the analyte is an antigen or hapten, reagent a) is a suspension of particles coated with the analyte or an analogue thereof, and reagent b) is an antibody to the analyte which antibody carries the saccharide.

4. A method as claimed in claim 3, wherein reagents b) and c) are reacted together prior to incubation with reagent a) and the sample.

5. A method as claimed in claim 1, wherein the analyte is an antigen, reagent a) is a suspension of particles coated with an antibody to the antigen which particles also carry the saccharide, and the assay is performed in two steps:

i) the sample is incubated with reagents a), b) and c) whereby particle agglutination take place,

ii) an excess of the saccharide is added to the incubation mixture,

and the extent of particle agglutination is thereafter observed.

6. A method of assay for bacteria in a sample by the use of particles coated with a glycoprotein or a saccharide, which method comprises incubating a suspension of the particles with the sample, in the presence of a lectin which binds to the glycoprotein or saccharide, and observing agglutination of the particles to an extent related to the bacterial concentration of the sample.

7. A method as claimed in claim 6, wherein the final step involves observing inhibition of particle agglutination caused by bacteria in the sample.

8. A method as claimed in claim 6, wherein the lectin is present on bacteria in the sample.

9. A method as claimed in any one of claims 1 to 8, wherein the extent of agglutination is determined by a particle counting technique.

| | **DOCUMENTS CONSIDERED TO BE RELEVANT** | | EP 89300100.8 |
|---|---|---|---|

| Category | Citation of document with indication, where appropriate, of relevant passages | Relevant to claim | CLASSIFICATION OF THE APPLICATION (Int. Cl.4) |
|---|---|---|---|
| A | EP - A2 - 0 166 623 (ORTHO DIA-GNOSTIC SYSTEMS INC.)<br><br>* Abstract; claims *<br><br>-- | 1-5 | G 01 N 33/566<br><br>G 01 N 33/569<br><br>G 01 N 33/545<br><br>C 12 Q 1/06<br><br>//C 12 Q 1/68 |
| A | WO - A1 - 86/03 839 (E.H.CERNY)<br><br>* Claims 1,4-8 *<br><br>-- | 1-5 | |
| A | EP - A2 - 0 106 685 (SYVA COMPANY)<br><br>* Pages 5-7 *<br><br>-- | 1-5 | |
| A | EP - A1 - 0 174 195 (THE WELLCOME FOUNDATION LIMITED)<br><br>* Page 4, lines 12-20; page 5, lines 9-15 *<br><br>---- | 6-9 | |

| | | | TECHNICAL FIELDS SEARCHED (Int. Cl.4) |
|---|---|---|---|
| | | | G 01 N 33/00<br><br>C 12 Q |

The present search report has been drawn up for all claims

| Place of search | Date of completion of the search | Examiner |
|---|---|---|
| VIENNA | 08-03-1989 | SCHNASS |